# EUROPÄISCHE PATENTSCHRIFT

(11) **EP 1 185 244 B1**
(45) Veröffentlichungstag und Bekanntmachung des Hinweises auf die Patenterteilung: **15.09.2004**
(21) Anmeldenummer: 00947797.7
(22) Anmeldetag: 08.06.2000
(51) Int. Cl.: A61K 7/48

(54) **ENZYMHALTIGES KOSMETIKUM**
COSMETIC PRODUCT CONTAINING ENZYMES
PRODUIT COSMETIQUE CONTENANT DES ENZYMES

(30) Priorität: 10.06.1999 DE 19927229
(43) Veröffentlichungstag der Anmeldung: 13.03.2002
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: PCT/DE2000/001891
(87) Internationale Veröffentlichungsnummer: WO 2000/076458

(56) Entgegenhaltungen:
- DATABASE CHEMABS [Online] CHEMICAL ABSTRACTS SERVICE, COLUMBUS, OHIO, US; KAWASAKI, YOSHIMI ET AL: "Bath preparations containing coconut milk" retrieved from STN Database accession no. 121:91319 CA XP002153316 & JP 05 331045 A (TSUMURA & CO, JAPAN) 14. Dezember 1993 (1993-12-14)
- DATABASE CHEMABS [Online] CHEMICAL ABSTRACTS SERVICE, COLUMBUS, OHIO, US; TANAKA, ISAMU, JAPAN: "Bath preparations" retrieved from STN Database accession no. 94:145189 CA XP002153317 & JP 56 007712 A (TANAKA, ISAMU, JAPAN) 27. Januar 1981 (1981-01-27)
- PATENT ABSTRACTS OF JAPAN vol. 011, no. 222 (C-435), 18. Juli 1987 (1987-07-18) & JP 62 036304 A (KASHIWA KAGAKU KOGYO:KK), 17. Februar 1987 (1987-02-17)
- A.Y. LEUNG: "Encyclopedia of common natural ingredients" 1996 , WILEY-INTERSCIENCE PUBLICATION XP002153315 Seite 299-300

## Beschreibung

Die Erfindung betrifft ein enzymhaltiges Kosmetikum mit intensiver Hautwirkung, insbesondere einer regenerativen Wirkung.

Es sind bereits eine Reihe von kosmetischen Erzeugnissen bekannt, in denen die Milch von Tieren oder ein Milchsäurederivat verarbeitet worden ist. So ist z.B. aus der DE-A-44 08 258 eine Vollmilch enthaltende Öl/Wasser Emulsion bekannt, die als Emulgator polyethoxyliertes Vitamin E enthält und eine Viskosität unter 100 Pa·s aufweist. Aus der DE-A-195 37 297 sind kosmetische Präparate mit Wachstumsfaktoren bekannt, hergestellt aus frischer oder pasteurisierter Milch und Kolostrum von Kühen und Stuten. Weiterhin ist aus der EP-A-908171 eine trockene pflanzliche Reinigungszusammensetzung bekannt, bei der unterschiedliche getrocknete und zerkleinerte Pflanzen als Detergenz, Schäummittel, Schaumbeschleuniger, Pigment und Konditionierungsmittel in einem Puder vereinigt werden, wobei Cocoa nucifera als Schaumbeschleuniger verwendet wird.

Der Erfindung liegt die Aufgabe zugrunde, ein besonders mild wirkendes Kosmetikum mit reizausgleichenden, regenerativen und dabei feuchtigkeitsregulierenden Eigenschaften bereitzustellen.

Erfindungsgemäß bereitgestellt wird ein enzymhaltiges Kosmetikum, gekennzeichnet durch einen Gehalt an
0,01 bis 5 Gew-% eines Konzentrates der Kokosmilch von Cocos nucifera, enthaltend Peroxidase, Lipase und Protease;
0,01 bis 10 Gew-% eines pflanzlichen Milchwassers, wobei die Pflanzen ausgewählt sind aus der Gruppe, die aus
Musa (Bananen), Taraxacum (Löwenzahn), Convolvus (Windengewächse), Papaver (Mohn), Soja und Gemischen davon besteht;
0,01 bis 5 Gew-% eines Glycerinextraktes eines Gemisches, bestehend aus Honig, Reisschalen, Reisschalenöl und/oder Reiskeimöl;
99,97 bis 80 Gew-% kosmetische Hilfsstoffe, weitere Wirkstoffe und Trägerstoffe.

Das erfindungsgemäße pflanzliche Milchwasser wird durch Auspressen der oberirdischen frischen Pflanzenteile (Stengel, Blätter, Blüten) gewonnen, vorzugsweise von Pflanzen nach Abschluß der Blüte, und es wird nach der Entfernung eventueller fester Bestandteile direkt in dem Kosmetikum eingesetzt. Unter dem Begriff "pflanzliches Milchwasser" sind die durch kaltes Auspressen erhältlichen, meist milchigen Pflanzensäfte zu verstehen. Ein bevorzugtes pflanzliches Milchwasser ist das von Löwenzahn und Soja.

Die in dem Kokosmilchkonzentrat enthaltenen Enzyme zeigen eine ausgeprägte Wirkung in mehrfacher Hinsicht. Mit Hilfe des durch Lipase bewirkten Abbaus von Fettsäuretriglyceriden zu Fettsäuren und Glycerin wird die Lipolyse dieser Stoffe beschleunigt, und ein verbesserter Austrag über das Blut ermöglicht. Zusammen mit Protease, das eine Schlüsselrolle bei der Hautregenerierung spielt, kann ein schnellerer Umschlag der Zellregenerierung erfolgen und infolge der proteolytischen Wirksamkeit kann die Zellerneuerung und die Wundheilung im dermalen Bereich unterstützt und beschleunigt werden.

Peroxidase hat radikalfangende Eigenschaften und tritt vermehrt in Pflanzen auf, die starkem äußerem Stress ausgesetzt sind, wie Ozon, Luftverschmutzung, Strahlung, Nährstoffveränderungen. Freie OH-Radikale und Peroxide werden durch die Peroxidase in Wasser und unschädliche Stoffe umgewandelt, wodurch eine erkennbare Anti-Alterungswirkung gegenüber der Hautzelle erreicht wird.

Das Koskosmilchkonzentrat, das auch durch Extraktion mit Propylenglycol gewonnen werden kann, kann weiterhin Cytokinin enthalten, welches infolge seiner Eigenschaft, die Zellteilung zu stimulieren, die Hautregenerierung vorteilhaft beeinflußt.

Insgesamt zeigen die drei in dem enzymhaltigen Kosmetikum der Erfindung vorliegenden Wirkstoffe eine Gesamtwirkung im Regenerierungsprozeß der Haut, die deutlich über der Einzelwirkung der Komponenten liegt. Besonders geeignet sind die Wirkstoffe daher in Regenerierungscremes, Anti-Alterungscremes und -lotionen, After-sun-Cremes, Schlankheitcremes und Festigungscremes.

Darüber hinaus zeigt das Kosmetikum sehr gute feuchtigkeitsregulierende, pH-regulierende und reizausgleichende Wirkungen, die mit den anderen Eigenschaften zu einem ausgeprägten Wohlempfinden führen.

Besonders hervorzuheben ist die Unterstützung des physiologischen Hydratisierungsprozesses der Haut durch das erfindungsgemäße Kosmetikum. Entsprechende Messungen mit dem Corneometer® zeigen eine deutliche langandauernde Wirkung, d.h. bei der Messung nach 8 Stunden war nur ein Rückgang der Feuchtigkeitszunahme nach dem Auftragen einer Lotion im Bereich von 4-12 % festzustellen.

Zu den kosmetischen Hilfs- und Trägerstoffe, wie sie üblicherweise in Zubereitungen wie dem vorliegenden Kosmetikum verwendet werden, gehören z.B. Wasser, Konservierungsmittel, Vitamine, Farbstoffe, Pigmente mit färbender Wirkung, Radikalfänger, Verdickungsmittel, weichmachende Substanzen, feuchthaltende Substanzen, Duftstoffe, Alkohole, Polyole, Elektrolyte, Ester, Gelbildner, polare und unpolare öle, Polymere, Copolymere, Emulgatoren, Wachse, Stabilisatoren.

Weitere Zusatz- bzw. Wirkstoffe in den kosmetischen Zusammensetzungen können sein Vitamine oder Vitaminkomplexe, z.B. Vitamin A oder Vitamin A-Derivate und/oder Vitamin E oder Vitamine-Derivate; weitere biogene Pflanzenextrakte, wie fettlöslicher Gardenienextrakt, fettlöslicher Karottenextrakt, Paprika-LS-Extrakt, β-Caroten, Lithospermum-Extrakt; organische Lichtschutzmittel wie z.B. Octylmethoxycinnamat; Methyl gluceth 10 oder Methyl gluceth 20; sowie aktive Deodorantien, wie Triclosan, oder Geruchslöscher, wie Grillocin®.

Zu den kosmetischen Wirkstoffen gehören weiterhin Emulgatoren, anorganische und organische Lichtschutzmittel, Radikalfänger, Enzyme, pflanzliche Wirkstoffe, Polymere, Melanin, Antioxidationsmittel, entzündungswidrige natürliche Wirkstoffe, mit Sauerstoff beladene asymmetrische lamellare Aggregate gemäß WO 94/00109; Aufschlußprodukte von Hefen oder pflanzlichen stoffen, hergestellt durch ein schonendes Ultraschall-Aufschlußverfahren gemäß WO 94/13783, Kaolin sowie mit SiO₂ modifiziertes Kaolin gemäß WO94/17588, eines im wäßrigen Medium mild abgebauten und mit einer unterstöchiometrischen Menge eines C₁₀-C₂₀-Fettsäurehalogenids kondensierten Produktes aus pflanzlichem oder tierischem Ausgangsmaterial gemäß der WO96/29048.

Als Ester oder Ether sind zum Beispiel geeignet (INCI-Namen): Dipentaerythrityl hexacaprilate/hexacaprate/tridecyl trimellitate/tridecyl stearate/neopentyl glycol dicaprylate dicaprate, Propylene glycol dioctanoate 5, Propylene glycol dicaprylate 2,30 dicaprate, Tridecyl stearate/neopentyl glycol dicaprylate dicaprate/tridecyl trimellitate, Neopentyl glycol dioctanoate, Isopropyle myristate, Diisopropyl dimer dilinoleate, Trimethylpropane triisostearate, Myristyl ether, Stearyl ether, Butyl ether, Dicaprylyl ether, PPG1-PEG9 Lauroyl glycol ether, PPG15 Stearyl ether, PPG14 Butyl ether, Fomblin® HC25.

Das enzymhaltige Kosmetikum kann als O/W- oder als W/O-Emulsion vorliegen. Die dafür eingesetzten öle können übliche kosmetische öle sein, wie ein Mineralöl; hydriertes Polyisobuten (INCI-Name: Hydrogenated polyisobutene); synthetisches oder aus Naturprodukten hergestelltes Squalan (INCI-Name: squalane, z.B. Synthesqual®, Cosbiol®); kosmetische Ester oder Ether, die verzweigt oder unverzweigt, gesättigt oder ungesättigt sein können; pflanzliche Öle; oder Gemische zweier oder mehrerer davon.

Besonders geeignet öle sind beispielsweise Mineralöle, Hydrogenated Polyisobuten, Polyisopren, squalane, Tridecyltrimellitat, Trimethylpropan-triisostearat, Isodecylcitrat, Neopentylglycol-diheptanoat, PPG-15-stearylether, Calendulaöl, Jojobaöl, Avocadoöl, Macadamianußöl, Rizinusöl oder ein Gemisch mehrerer davon. Je nachdem welche Öle ausgewählt werden, werden die kosmetischen Eigenschaften der festen Zusammensetzung beeinflußt, wie Transparenzgrad, Weichheit, Härte, Spreitungswirkung.

Das Kosmetikum kann auch als Gel vorliegen. Zu geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, bestimmte Polyacrylate, Polyvinylalkohol, Polyvinylpyrrolidon, Montmorillonit etc.

In dem erfindungsgemäßen Kosmetikum können als Erweichungsmittel normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, oleylalkohol, Isopropyllaurat, Decyloleat, Octadecan-2-ol, Isocetylalkohol, Cetylpalmitat, Siliconöle wie Dimethylpolysiloxan, Isopropylmyristat, Isopropylpalmitat, Polyethylenglycol, Lanolin, Kakaobutter, pflanzliche öle wie Maisöl, Baumwollsamenöl, Olivenöl, mineralische öle, Butylmyristat, Palmitinsäure usw.

Zu Antioxidationsmitteln, die in der Erfindung verwendet werden können, gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B α-Carotin, β-Carotin; Harnsäure und Derivate davon; α-Hydroxysäuren wie Citronensäure, Milchsäure, Apfelsäure; Stilbene und deren Derivat usw.

In einer bevorzugten Ausführungsform der Erfindung enthält das enzymhaltige Kosmetikum 0,01 bis 2 Gew-% Milchsäure. Dadurch kann die Penetrierung anderer Wirkstoffe in die Haut deutlich verbessert werden.

Vorteilhaft für die erfindungsgemäßen Zubereitungen kann es sein, daß das Kosmetikum verkapselt in asymmetrischen lamellaren Aggregaten enthält, wobei diese Aggregate aus Phospholipiden und mit Sauerstoff beladenes Fluorcarbon oder Fluorcarbongemisch bestehen und deren Gehalt an Fluorcarbon im Bereich von 0,2 bis 100 % Gewicht/Volumen liegt, wobei das Phospholipid einen Phosphatidylcholingehalt von mehr als 30 bis 99 Gewichts-% hat, und wobei diese Aggregate eine Hautpenetrierung in Abhängigkeit von der kritischen Löslichkeitstemperatur der Fluorcarbone besitzen.

Der Gehalt dieser Aggregate kann im Bereich von 0,05 bis 20 Gew-% liegen, bezogen auf die Gesamtmasse des Kosmetikums.

Die Aggregate können auch zusätzlich allein nur mit Sauerstoff beladen in der kosmetischen Zubereitung vorliegen.

Diese Aggregate sind Sauerstoffträger und ermöglichen ein Penetrieren des Sauerstoffs in die Haut und damit eine bessere Versorgung der Haut mit Sauerstoff. Die Herstellung dieser Aggregate erfolgt durch Hochdruckhomogenisierung von Phospholipiden, wie Sojalecithin und Eilecithin oder synthetischen Phospholipiden oder teilhydrierten Phospholipiden, die einen Phosphatidylcholingehalt von mehr als 30 Gew.-% bis 99 Gew.-% haben, mit perfluorierten oder hochfluorierten Kohlenstoffverbindungen oder Gemischen davon, die in der Lage sind, Gase, wie Sauerstoff und Kohlendioxid zu transportieren. Darin können neben Phosphatidylcholin auch Lysolecithine im Konzentrationsbereich von 0,1 bis 10 Gew.-% und/oder geladene Phospholipide wie Phosphatidylethanolamin, n-Acetylphosphatidylethanolamin oder Phosphatidsäure im Konzentrationsbereich 0,1 bis 30 Gew.-% vorhanden sein.

Im Unterschied zu den bekannten wäßrigen Liposomen (Vesikel) tragen diese Phospholipid-stabilisierten Aggregate in ihrem Kern hydrophobe Fluorcarbone, die zum Transport von Sauerstoff befähigt sind. Ihre grenzflächenchemische Stabilisierung erfolgt primär durch eine Monolayer mit inverser Anordnung und gegebenenfalls ein sich daran anschließender Aufbau von Bilayer-Schichten. Wegen der Besonderheit ihrer strukturellen Anordnung werden diese neuartigen Aggregate als asymmetrische lamellare Sauerstoff-Carrier bezeichnet. Ihre außergewöhnliche kolloidchemische Stabilität ist vermutlich auf die lamellare Struktur und auf die Oberflächenladung der Aggregate zurückzuführen. Letztere ist auf die Auswahl geeigneter Phospholipide beziehungsweise deren Mischungen natürlicher wie auch synthetischer Provenienz zurückzuführen. In erster Linie sind für eine vorteilhafte Wirkung in diesem Sinne Phospholipide, insbesondere Phosphatidylcholin im genannten Konzentrationsbereich von 30 bis 99 % in Verbindung mit Lysolecithinen der Konzentration von 0,1 bis 10 % und/oder geladenen Phospholipiden im Konzentrationsbereich 0,1 bis 30 Gew.-% verantwortlich. Die angesprochene Wirkung der Phospholipide wird durch entsprechende negative Zeta-Potentiale und durch die Messung von Ladungsdichten (bei Titration mit einem kationischen Polyelektrolyten) verifiziert. Wesentlich für den Einsatz der Fluorcarbon-Aggregate ist die Hautpenetrierung in Abhängigkeit von der kritischen Löslichkeitstemperatur der ausgewählten Fluorcarbone oder Fluorcarbongemische (für den Einsatz asymmetrischer lamellarer Aggregate s.a. DE-C-42 21 255).

Einen weiteren Wirkstoff, den das erfindungsgemäße Kosmetikum enthalten kann, sind fein verteilte hartmagnetische Einbereichsteilchen (Einkristalle) mit hoher Koerzitivfeldstärke und mit Korngrößen im Bereich von 100 bis 1200 nm mit oder ohne die o.g. asymmetrischen lamellaren Aggregate, wobei diese hartmagnetischen Teilchen insbesondere Barium- und/oder Strontiumhexaferrite sind, erzeugt nach der Glaskristallisationstechnik durch Züchtung von Einkristallen aus einer abgeschreckten Glasschmelze (siehe WO95/03061 und WO98/44895).

Als weiteren Wirkstoff kann das Kosmetikum vorteilhaft Kaolin gemäß WO96/17588 enthalten, der mit sphärischen TiO₂- oder SiO₂-Teilchen mit einer Teilchengröße <5 µm modifiziert ist, wobei die sphärischen Teilchen einen Anteil an der Kaolinmischung von 0,5 bis 10 Gew-% haben. Das Präparat erhält dadurch ein sehr weiches Hautgefühl und eine zusätzliche entzündungswidrige Wirksamkeit.

Der modifizierte Kaolin kann einen Anteil von 0,1 bis 6 Gew-% haben, bezogen auf die Gesamtmenge des Kosmetikums.

Das Kosmetikum kann auch Pigmente, Pigmentgemische oder Pulver mit pigmentartiger Wirkung enthalten, worunter auch solche mit Perlglanz-Effekt zu verstehen sind, können zum Beispiel umfassen Eisenoxide, Titan(di)oxid, Glimmer, Kaolin, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, verkapselte und unverkapselte Getreidestärken sowie Glimmer-Titanoxid-organischer Farbstoff.

Es ist weiterhin vorteilhaft, den erfindungsgemäßen Zusamensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder salze wie das Naoder K-Salz der 2-Phenmylbenzimidazol-5-sulfonsäure.

Zu UVA-Filtern gehören Dibenzoylmethan-Derivate wie 1-Phenyl-4-(4'-isopropylphenyl)propan-1,3-dion.

Bevorzugt als Sonnenschutzfilter sind anorganische Pigmente auf Basis von Metalloxiden, wie TiO₂, SiO₂, ZnO, Fe₂O₃, ZrO₂, MnO, Al₂O₃, die auch im Gemisch eingesetzt werden können.

Das Kosmetikum kann auch einen Gehalt an Schellack in der wäßrigen Phase aufweisen, wobei der Anteil an reinem Schellack in einer O/W-Emulsion im Bereich von 0,1 bis 20 Gew-%, in einer W/O-Emulsion im Bereich von 0,1 bis 15 Gew-%, in einem Hydrogel im Bereich von 0,1 bis 10 Gew-% liegt. Die Herstellung dieses wasserlöslichen Schellacks erfolgt gemäß der WO99/06011 bzw. WO99/06488.

Ein weiterer Zusatz für das erfindungsgemäße Kosmetikum ist eine Wirkstoffkombination aus a) einem Produkt eines enzymatischen Extraktionsprozesses des maritimen Planktons Artemia salina, wobei das Extraktionsprodukt aus phosphorylierten Nucleotiden mit der Hauptkomponente Diguanosin-tetraphosphat besteht; b) D-myo-Inosit-1,4,5-triphosphat; c) Glycan; wobei das Verhältnis a:b:c im Bereich 1 : 0,1-50 : 0,1-30 liegt. Diese Kombination zeigt einen Synergie-Effekt gegen aggressive Umwelteinflüsse durch natürliche Verstärkung des Immunsystems der Haut, Stimulierung der Hautregenerierung und zugleich UV-Schutz durch die verbesserte Keratin-Barriere.

Ein weiterer Zusatz für das erfindungsgemäße Kosmetikum ist eine Wirkstoffzubereitung mit hohem Radikalschutzfaktor mit einem Gehalt an einem durch Extraktion der Rinde von Quebracho blanco und nachfolgender enzymatischer Hydrolyse gewonnenem Produkt, das wenigstens 90 Gew- % Proanthocyanidin-Oligomere und höchstens 10 Gew-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser.

Das für die Erfindung eingesetzte Konzentrat der Kokosmilch von Cocos nucifera ist ein Handelsprodukt, das unter dem Namen Cocozyme® von Greentech S.A, St. Beauzire Cedex, Frankreich angeboten wird. Das Kokosmilchkonzentrat ist eine hellgelbe, in Wasser und Alkohol lösliche Flüssigkeit.

1 kg des oben genannten Glycerinextraktes werden aus etwa 5g Honig, 50 g Reisschalen und 10 g Reiskeimöl oder Reisschalenöl hergestellt (Extrapone Honey/Rice Blend GW® von Dragoco, Holzminden, Deutschland sowie Extrapone Honey Rice Milk®).

Die Verwendung des erfindungsgemäßen kosmetischen Präparates kann z.B. erfolgen in Sonnencremes, Sonnengelen, After-sun-Produkten, Tagescremes, Nachtcremes, Masken, Körperlotionen, Reinigungsmilch, Make up's, Körperpuder, Augenkosmetik, Haarmasken, Haarspülungen, Haarshampoos, Duschgelen, Duschölen, Badeölen. Die Herstellung derartiger Produkte erfolgt auf eine Weise, wie sie dem Fachmann auf diesem Gebiet bekannt ist.

Die Erfindung soll nachstehend durch Beispiele näher erläutert werden. Alle Angaben erfolgen in Gewichtsprozent, sofern nichts anderes angegeben ist.

| Beispiel 1 **Feuchtigkeitscreme für alle Hauttypen** | |
|---|---|
| **Phase A** | |
| Steareth-2 | 2,5 |
| Steareth-21 | 1,5 |
| Beeswax | 6,0 |
| Jojoba Oil | 1,5 |

| **Phase B** | |
|---|---|
| Wasser | q.s. ad 100 |
| Copolymer | 0,5 |
| Glycerin | 3,0 |

| **Phase C** | |
|---|---|
| Triethanolamin | 0,5 |

| **Phase D** | |
|---|---|
| Konservierungsmittel | 0,5 |

| **Phase E** | |
|---|---|
| Kokosmilchkonzentrat | |
| Coconut(Cocos nicifera)Water | 0,5 |
| pflanzliches Milchwasser aus | |
| Sojapflanzen (Glycine spp.) | 1,0 |
| Glycerinextrakt aus Honig, | |
| Reisschalen und Reisschalenöl | |
| Water&Glycerin&Rice (Oryza sativa)Bran | |
| Extract&PEG-40 Hydrogenated Castor Oil | |
| &Trideceth-9&Rice(Oryza sativa)Bran Oil | |
| & Honey Extract | 0,5 |

| **Phase F** | |
|---|---|
| Parfüm | 0,2 |

Die Herstellung dieser Creme erfolgt in der Weise, daß die Phasen A und B separat auf 60 bis 70 °C erwärmt werden, danach die Phase A in der Phase B verrührt wird, und beide Phasen und gut miteinander homogenisiert werden. Anschließend wird die Phase C zugegeben und kurz homogenisiert. Es wird auf etwa 40 °C abgekühlt, und nacheinander werden die Phasen D bis F in dieser Reihenfolge unter Rühren zugegeben

| Beispiel 2 **Shampoo und Gel 2-in-1** | |
|---|---|
| Wasser | q.s. ad 100 |
| Sodium Laureth Sulfate | 53,0 |
| Disodium EDTA | 0,1 |
| Polyquaternium-7 | 1,0 |
| Panthenol | 0,1 |
| Konservierungsmittel | 0,3 |
| Kokosmilchkonzentrat | 5,0 |
| pflanzliches Milchwasser aus Löwenzahn (Taraxum spp.) | 10,0 |
| Glycerinextrakt aus Honig, | |
| Reisschalen und Reiskeimöl | 3,0 |

Die genannten Substanzen werden bei Raumtemperatur nacheinander zu einer homogenen Mischung verrührt.

| Beispiel 3 **Feuchtigkeitscreme mit langandauernder Wirkung** | |
|---|---|
| **Phase A** | |
| Steareth 2 | 2,0 |
| Steareth 21 | 2,21 |
| Stearic Acid | 1,8 |
| Stearyl Alcohol | 3,2 |

| **Phase B** | |
|---|---|
| Wasser | q.s. ad 100 |
| Glycerin | 5,0 |
| modifiziertes Kaolin mit SiO₂* | 5,0 |

| **Phase C** | |
|---|---|
| asymmetrischen lamellaren Aggregate** | 10,0 |
| pflanzliches Milchwasser aus Löwenzahn | |
| (Taraxum spp.) | 2,0 |
| pflanzliches Milchwasser aus | |
| Sojapflanzen (Glycine spp.) | 1,5 |
| Glycerinextrakt aus Honig, | |
| Reisschalen und Reiskeimöl | 1,0 |
| Kokosmilchkonzentrat | 1,0 |
| RPF-Komplex*** | 1,5 |

| **Phase D** | |
|---|---|
| Konservierungsmittel | 1,0 |

| **Phase E** | |
|---|---|
| Parfüm | q.s. |

| | |
|---|---|
| Die Phasen A und B werden separat auf etwa 65°C erwärmt und unter Rühren miteinander vermischt. Nach dem Abkühlen auf etwa 40 °C unter Rühren werden die Phasen C, E und F ebenfalls unter Rühren zugegeben. * Kaolin gemäß WO96/17588; ** Aggregate gemäß WO94/00109 ; | |
| *** Wirkstoffkomplex mit hohem Radikalschutzfaktor gemäß WO99/66881 | |

| Beispiel 4 **Gel für Gesicht und Körper mit SPF 15** | |
|---|---|
| **Phase A** | |
| Wasser | q.s. ad 100 |
| Crosspolymer | 0,1 |
| Glycerin | 2,0 |

| **Phase B** | |
|---|---|
| Triethanolamin | 0,1 |

| **Phase C** | |
|---|---|
| Octyl Methoxycinnamate | 7,5 |
| Octyl Salicylate | 5,0 |
| Butyl Methoxydibenzoylmenthane | 3,0 |

| **Phase D** | |
|---|---|
| Stabileze QM PVM/MA Decadiene Crosspolymer | 1,5 |

| **Phase E** | |
|---|---|
| Shellac* | 5 |
| modifiziertes Kaolin mit SiO₂** | 1,5 |
| RPF-Komplex*** | |
| Sun Marine Komplex⁺ | 1,0 |
| Glycerinextrakt aus Honig, | |
| Reisschalen und Reiskeimöl | 0,5 |
| Kokosmilchkonzentrat | 0,5 |
| pflanzliches Milchwasser aus Löwenzahn | |
| (Taraxum spp.) | 0,5 |
| Milchsäure | 0,4 |
| Parfüm | q.s. |
| Konservierungsmittel | 1,0 |

| | |
|---|---|
| Die Phasen wurden bei Raumtemperatur nacheinander in der angegebenen Reihenfolge vermischt und gut homogenisiert. * Schellack gemäß WO99/06011 Beispiel 2; | |
| ** Kaolin gemäß WO96/17588; | |
| *** Wirkstoffkomplex mit hohem Radikalschutzfaktor gemäß WO99/66881; ⁺ Hefeaufschlußprodukt gemäß WO98/25584 Beispiel 1. | |

## Patentansprüche

1. Enzymhaltiges Kosmetikum, **gekennzeichnet durch** einen Gehalt an 0,01 bis 5 Gew-% eines Konzentrates der Kokosmilch von Cocos nucifera, enthaltend Peroxidase, Lipase und Protease;
0,01 bis 10 Gew-% eines pflanzlichen Milchwassers, wobei die Pflanzen ausgewählt sind aus der Gruppe, die aus
Musa (Bananen), Taraxacum (Löwenzahn), Convolvus (Windengewächse), Papaver (Mohn), Glycine (Soja) und Gemischen davon besteht; 0,01 bis 5 Gew-% eines Glycerinextraktes eines Gemisches, bestehend aus Honig, Reisschalen, Reisschalenöl und/oder Reiskeimöl;
99,97 bis 78 Gew-% kosmetische Hilfsstoffe, Wirkstoffe und Trägerstoffe.

2. Enzymhaltiges Kosmetikum nach Anspruch 1, **dadurch gekennzeichnet, daß** es 0,01 bis 2 Gew-% Milchsäure enthält.

3. Enzymhaltiges Kosmetikum nach Anspruch 1, **dadurch gekennzeichnet, daß** das pflanzliche Milchwasser durch Kaltpressen der oberirdischen Pflanzenteile erhalten wird.

4. Enzymhaltiges Kosmetikum nach Anspruch 1, **dadurch gekennzeichnet, daß**. das pflanzliche Milchwasser ein Milchwasser von Löwenzahn oder Soja oder deren Gemischen ist.

5. Verwendung eines Gemisches gemäß Anspruch 1 oder 2 in kosmetischen Mitteln zur Hautregenerierung.

## Claims

1. A cosmetic product containing enzymes, **characterized in that** it contains
0.01 to 5 % by weight of a concentrate of the coconut milk of *Cocos nucifera*, containing peroxidase, lipase and protease;
0.01 to 10 % by weight of a plant milkwater, the plants being selected from the group consisting of *Musa* (bananas), *Taraxacum* (dandelion), *Convolvulus (Convolvulaceae),* Papaver (poppy), *Glycine* (soya) and mixtures thereof;
0.01 to 5 % by weight of a glycerol extract of a mixture consisting of honey, rice hulls, rice hull oil and/or rice germ oil;
99.97 to 78 % by weight of cosmetic auxiliary agents, active agents and carrier substances.

2. A cosmetic product containing enzymes according to Claim 1, **characterized in that** it contains 0.01 to 2 % by weight of lactic acid.

3. A cosmetic product containing enzymes according to Claim 1, **characterized in that** the plant milkwater is obtained by cold pressing the overground parts of the plants.

4. A cosmetic product containing enzymes according to Claim 1, **characterized in that** the plant milkwater is a milkwater of dandelion or soya, or a mixture thereof.

5. The use of a mixture according to Claim 1 or 2 in cosmetic preparations intended for skin regeneration.

## Revendications

1. Produit cosmétique contenant des enzymes, **caractérisé par** une teneur allant de 0,01 à 5 % en poids d'un concentré de lait de coco de *Cocos nucifera*, contenant des peroxydases, lipases et protéases ; de 0,01 à 10 % en poids d'une eau laiteuse végétale, où les plantes sont choisies parmi le groupe qui consiste en Musa (bananes), *Taraxacum* (pissenlit), *Convolvus* (liseron), Papaver (pavot), *Glycine* (soya) et des mélanges de ceux-ci ; de 0,01 à 5 % en poids d'un extrait glycérinique d'un mélange consistant en du miel, de son de riz, de l'huile de son de riz et/ou de l'huile de germe de riz ; de 99,97 à 78 % en poids d'auxiliaires cosmétiques, d'agents actifs et de matières supports.

2. Produit cosmétique contenant des enzymes selon la revendication 1, **caractérisé en ce qu'**il contient 0,01 à 2 % en poids d'acide lactique.

3. Produit cosmétique contenant des enzymes selon la revendication 1, **caractérisé en ce que** l'eau laiteuse végétale est obtenue par pressage à froid des parties aériennes des plantes.

4. Produit cosmétique contenant des enzymes selon la revendication 1, **caractérisé en ce que** l'eau laiteuse végétale est une eau laiteuse de pissenlit ou de soya ou leurs mélanges.

5. Utilisation d'un mélange selon la revendication 1 ou 2 dans des agents cosmétiques pour la régénération de la peau.
